# EUROPÄISCHE PATENTSCHRIFT

(11) **EP 1 890 743 B1**
(45) Veröffentlichungstag und Bekanntmachung des Hinweises auf die Patenterteilung: **30.01.2019**
(21) Anmeldenummer: 06742699.9
(22) Anmeldetag: 26.04.2006
(51) Int. Cl.: A61B 5/145, A61M 5/142, A61B 5/00, G06F 19/00

(54) **ENERGIEOPTIMIERTE DATENÜBERTRAGUNG EINES MEDIZINISCHEN GERÄTS**
ENERGY-OPTIMISED DATA TRANSMISSION FOR A MEDICAL APPLIANCE
TRANSMISSION DE DONNEES A ENERGIE OPTIMISEE POUR UN APPAREIL MEDICAL

(30) Priorität: 26.04.2005 DE 102005019306
(43) Veröffentlichungstag der Anmeldung: 27.02.2008
(73) Patentinhaber: F. Hoffmann-La Roche AG, 4070 Basel (CH); Roche Diabetes Care GmbH, 68305 Mannheim (DE)
(72) Erfinder: SCHOEMAKER, Michael, 68183 Mannheim (DE); FRANKHAUSER, Sybille, 4542 Luterbach (CH); BERNINI, Nicole, 4107 Ettigen (CH); JECKELMANN, Joel, 1752 Villars-sur-Glâne (CH); LA BASTIDE, Sebastiaan, Oro Valley, CA 85755 (US); MEYER OLDEN, Gunnar, 26180 Rastede (DE); ESSENPREIS, Matthias, 3400 Burgdorf (CH)
(74) Vertreter: Rentsch Partner AG
(86) Internationale Anmeldenummer: PCT/EP2006/003869
(87) Internationale Veröffentlichungsnummer: WO 2006/114297

(56) Entgegenhaltungen:
- EP-A- 1 343 112
- US-A1- 2002 072 784
- US-A1- 2003 119 568
- US-A1- 2004 204 744
- US-A1- 2005 065 464
- US-B1- 6 239 724

## Beschreibung

Die Erfindung bezieht sich auf ein medizinisches Gerät, ein System zur energieoptimierten Datenübertragung von und/oder zu dem medizinischen Gerät.

Während des Betriebes eines medizinischen Gerätes, wie zum Beispiel einer auf der Haut eines Benutzers angebrachten Infusionspumpe oder eines Sensors, ist es für einen Benutzer von Vorteil, wenn dem medizinischen Gerät zum Beispiel von einer Fernsteuerung Daten für den Betrieb des medizinischen Gerätes übertragen werden können oder wenn zum Beispiel Sensor-Messdaten an ein externes Gerät übertragen werden.

Ein von einer Fernsteuerung ansteuerbares externes Infusionsgerät ist aus der US 6,551,276 B1 bekannt, wobei das externe Infusionsgerät einen Empfänger aufweist, welcher in einem so genannten Standby-Modus sein kann und automatisch ungefähr alle 2,5 Sekunden aktiviert wird, um festzustellen, ob von der Fernsteuerung ein Funksignal ausgesandt wird, sodass die Funkfernsteuerung ein Aktivierungssignal über eine Periode von zum Beispiel ca. 5 Sekunden aussenden sollte, um von dem Empfänger erkannt zu werden.

Die US 4,395,259 beschreibt ein implantierbares Messgerät, welches eine Einheit zur Abgabe einer Dosis aufweist, wobei ein externes Steuergerät induktiv mit diesem Gerät gekoppelt werden kann, um zum Beispiel die Infusionsrate zu verändern.

Die US 2004/204744 zeigt ein erstes Implantat mit einem ersten akustischen Transducer und ein zweites Implantat mit einem Schalter und einem zweiten akustischen Transducer. Der zweite akustische Transducer empfängt akustische Signale vom ersten akustischen Transducer, wobei ein Schalter geschlossen wird und das zweite Implantat aktiviert wird.

Die US 2003/0119568 zeigt einen Transceiver eines schlafenden Geräts, welcher eingerichtet ist, von einem Wake-up Receiver des schlafenden Geräts eingeschaltet zu werden, falls der Wake-up Receiver ein Wake-up Signal von einem aktiven Gerät empfängt. In einer Variante erhält der Wake-up Receiver Energie aus dem Wake-up Signal. Das aktive Gerät kann als Pager, Mobilfunkgerät oder Handheld ausgeführt sein. Das schlafende Gerät kann in einem Körper implantiert als Herzmonitor/Defibrillator ausgeführt sein, wobei das aktive Gerät Daten wie Batteriestand oder elektrokardiologische Signale ausliest oder andere Daten austauscht.

Es ist eine Aufgabe der vorliegenden Erfindung ein medizinisches Gerät, ein System vorzuschlagen, welche eine Datenübertragung bei einem geringen Energieeinsatz ermöglichen.

Diese Aufgabe wird durch die Gegenstände des unabhängiges Anspruchs gelöst.

Vorteilhafte Ausführungsformen ergeben sich aus den abhängigen Ansprüchen.

Ein medizinisches Gerät, wie zum Beispiel ein zum Beispiel subkutaner Sensor, insbesondere ein Glukose-Sensor, eine Pumpe, insbesondere eine Insulin-Pumpe, welche zum Beispiel implantiert, minimal invasiv oder nicht invasiv sein können, oder ein Pen zum Einsatz am und/oder im Körper eines Benutzers weist bevorzugt mindestens eine elektrische oder elektronische Funktionseinheit auf, wie zum Beispiel einen Messsensor, einen Prozessor, einen Speicher oder einen Elektromotor, welcher beispielsweise von dem Prozessor angesteuert werden kann und weist eine Sende- und/oder Empfangseinheit zum Übertragen, also zum Senden und/oder Empfangen von Daten auf, wobei erfindungsgemäß ein Aktivierungsschalter vorgesehen ist, der mit der Sende-/Empfangseinheit und/oder mindestens einer elektronischen Funktionseinheit in Verbindung steht, wobei der Aktivierungsschalter durch ein externes Aktivierungssignal aktiviert werden kann, um zum Beispiel die Sende-/Empfangseinheit und/oder mindestens eine elektronische Funktionseinheit zu aktivieren, sodass beispielsweise eine drahtlose Kommunikationsverbindung hergestellt werden kann, um zum Beispiel Messdaten von einem Sensor an ein externes Gerät zu übertragen oder zum Beispiel Steuerdaten zur Einstellung einer Basalrate oder zur Abgabe eines Bolus an eine Insulin-Pumpe zu senden oder die durch die Kommunikationsverbindungen verbundenen Geräte zu synchronisieren. Erfindungsgemäß wird die Kommunikation nur bei Bedarf, also "on demand" aufgenommen, wobei durch die für die Datenübertragung erforderliche Aktivierung des Aktivierungsschalters sichergestellt werden kann, dass nur dann eine Kommunikation aufgebaut oder hergestellt wird, wenn Empfänger und Sender, also das medizinische Gerät und ein externes Gerät, wie zum Beispiel eine Steuervorrichtung oder die jeweiligen Eingabe-/Ausgabeeinheiten davon, entweder in direktem physikalischen Kontakt miteinander stehen oder so relativ zueinander angeordnet sind, dass eine vorgegebene maximale Entfernung zwischen Sender und Empfänger unterschritten wird. Weiterhin kann zum Beispiel der Aktivierungsschalter des medizinischen Gerätes, welcher von einem Aktivierungselement des externen Gerätes aktiviert wird, nur dann ein Anschalt- oder Freigabesignal ausgeben, wenn das medizinische Gerät und ein externes Gerät eine vorgegebene Orientierung zueinander haben, also zum Beispiel Sende- und Empfangseinheiten oder Sende- und Empfangselemente etwa einander gegenüber liegen. Somit kann der Energiebedarf des medizinischen Gerätes verringert werden, da nur dann elektrische oder elektronische Komponenten zur Übertragung von Daten aktiviert werden, wenn ein entsprechender Sender oder Empfänger eines externen Geräts in Reichweite ist, wobei die Sende- und Empfangseinheiten so dimensioniert oder ausgelegt werden können, dass eine Datenübertragung nur über eine relativ kurze Strecke, wie zum Beispiel 5 cm oder weniger, sichergestellt werden muss, was beispielsweise ausreicht, um Daten von einem externen Gerät, welche auf die Kleidung eines Benutzers aufgelegt wurde, durch diese Kleidung hindurch zu einem darunter auf der Haut des Patienten angebrachten oder implantierten medizinischen Gerätes zu übertragen oder von diesem medizinischen Gerät zu empfangen. Da die Datenübertragungseinheit erfindungsgemäß nur eine relativ geringe Leistungsfähigkeit aufweisen muss, kann diese im Vergleich zu zum Beispiel Datenübertragungseinheiten, welche für Entfernungen im Meterbereich ausgelegt sind, relativ klein ausgestaltet werden, wobei Sende- und Empfangs-Elemente, wie zum Beispiel Antennen, Induktionsspulen, oder andere elektromagnetische Wellen aussendende oder empfangende Elemente, wie zum Beispiel Lampen- oder Fotodioden oder Lautsprecher und Mikrophone nur eine geringe Reichweite und Leistungsfähigkeit besitzen müssen.

Der Aktivierungsschalter kann zum Beispiel auch ein Element oder Bauteil sein, mit welchem eine zum Beispiel von dem externen Gerät zur Verfügung gestellte Energie empfangen oder aufgenommen werden kann, um zum Beispiel mit dem externen Gerät kommunizieren zu können oder um eine zum Beispiel von dem externen Gerät vorgegebene Aktion auszuführen, so dass das medizinische Gerät in diesem Fall keine eigene Energieversorgung aufweisen muss, da diesem die benötigte Energie von dem externen Gerät zur Verfügung gestellt wird. Beispielsweise kann eine Induktionsspule als Aktivierungsschalter vorgesehen sein, wobei ein sich in der Nähe der Induktionsspule befindendes externes Gerät zum Beispiel ebenfalls eine Induktionsspule aufweisen kann, um über die induktive Kopplung der Induktionsspulen Energie an das medizinische Gerät zu übertragen, wodurch dieses aktiviert werden und zum Beispiel eine Messung durchführen kann.

Alternativ oder ergänzend kann ein Aktivierungsschalter auch als Magnetschalter, zum Beispiel als Reed-Kontakt ausgebildet sein, welcher von einem externen Magnetfeld, also zum Beispiel einem an dem externen Gerät vorgesehenen Permanent-Magneten oder Elektro-Magneten aktiviert wird und zum Beispiel eine Verbindung der in dem medizinischen Gerät vorhandenen elektrischen oder elektronischen Komponenten, wie zum Beispiel der Sende- / Empfangseinheit mit einer ebenfalls in dem medizinischen Gerät vorhandenen Energiequelle zum Beispiel in Form einer Batterie oder eines Akkumulators herstellt. Weiterhin können andere Sensoren, welche zum Beispiel elektromagnetische Felder, Schall oder Licht detektieren können, oder magnetische oder kapazitive Effekte nutzen, als Aktivierungsschalter verwendet werden, wie zum Beispiel eine Fotodiode oder ein Mikrophon.

Dabei kann der Aktivierungsschalter so ausgebildet sein, dass eine nach der Aktivierung durch ein externes Gerät hergestellte Kommunikationsverbindung wieder unterbrochen wird, wenn kein Aktivierungssignal mehr anliegt, also zum Beispiel wenn ein vorgegebener Mindestabstand zum externen Gerät wieder überschritten wird. Alternativ oder ergänzend kann die Aktivierung des medizinischen Gerätes auch nur für einen vorgegebenen Zeitraum erfolgen, welcher zum Beispiel benötigt wird, um Daten zu übertragen oder eine vorgegebene Aktion, wie zum Beispiel das Abgeben eines Bolus und/oder das Aussenden eines Bestätigungssignals, auszuführen.

Ebenso kann auch ein mechanischer Schalter als Aktivierungsschalter zum Beispiel unter einem gummiartigen Oberflächenelement vorgesehen sein, welcher zum Beispiel die Stromversorgung oder eine Sende-/Empfangseinheit des medizinischen Gerätes aktiviert, nachdem ein mechanischer Schaltvorgang, also zum Beispiel ein Druck auf den mechanischen Schalter, durch ein auf das medizinische Gerät aufgelegtes externes Gerät ausgelöst wurde. Dabei kann das medizinische Gerät beispielsweise ein Oberflächenprofil aufweisen, welches einem Gegenprofil des externen Gerätes entspricht, sodass das externe Gerät zum Beispiel in einer durch das Oberflächenprofil bestimmten Orientierung auf das medizinische Gerät aufgelegt werden muss, um dieses zu aktivieren oder um eine Datenverbindung herzustellen. Im Falle eines direkten Kontaktes zwischen dem medizinischen Gerät und dem externen Gerät kann eine Datenverbindung oder auch eine Energieübertragung zum Beispiel auch über eine elektrische Leitung erfolgen, wenn sich einander entsprechende Kontaktelemente des medizinischen Geräts und des externen Gerätes berühren.

Die Erfindung bezieht sich weiterhin auf ein Steuergerät, wie zum Beispiel eine Fernbedienung oder ein Blutglukose-Messgerät zum Ansteuern eines wie oben beschriebenen medizinischen Gerätes, wobei das Steuergerät als externes Gerät eine Aktivierungseinheit aufweist, mit welcher ein oder mehrere Aktivierungsschalter des medizinischen Gerätes aktiviert oder getätigt werden können. Als Aktivierungseinheit kann am externen Gerät zum Beispiel ein Magnet, insbesondere ein Permanentmagnet oder ein Elektromagnet, eine Induktionsspule zum Übertragen von Energie zu dem medizinischen Gerät, ein Lautsprecher, eine Lampe oder ein bestimmtes Oberflächenprofil zur Betätigung eines mechanischen Schalters vorgesehen sein. Weiterhin weist das Steuergerät eine Sende- und/oder Empfangseinheit auf, um eine Datenverbindung mit dem medizinischen Gerät herstellen zu können, wobei die Sende-/Empfangseinheit zum Beispiel aktiviert werden kann, nachdem ein Aktivierungssignal von der Aktivierungseinheit des Steuergerätes ausgesandt wurde oder vom Steuergerät detektiert wurde, dass eine Verbindung, wie zum Beispiel ein mechanischer Kontakt, eine Funkverbindung und/oder eine induktive Kopplung, mit dem medizinischen Gerät hergestellt wurde, oder besteht.

Das externe Gerät kann eine Eingabeeinheit, zum Beispiel eine Tastatur oder einen Touch-Screen, aufweisen, um es einem Benutzer zu ermöglichen Steuersignale in das Steuergerät einzugeben, welche von diesem an das medizinische Gerät übertragen werden können, um zum Beispiel die Arbeitsweise einer implantierten Insulinpumpe zu steuern, wie zum Beispiel die Basalrate oder einen Bolus einzustellen. Vorzugsweise weist das Steuergerät eine Anzeigeeinheit oder ein Display auf, an welchem zum Beispiel zu übertragende Steuerdaten, von einem Benutzer eingegebene Befehle oder Parameter, oder von dem medizinischen Gerät empfangene Daten, wie zum Beispiel Messdaten eines Sensors, dargestellt werden können.

Bevorzugt weist das Steuergerät eine Schnittstelle für die Kommunikation mit einem anderen elektrischen Gerät auf, wie zum Beispiel eine USB-Schnittstelle, eine Infrarot-Schnittstelle oder eine Bluetooth-Schnittstelle, um Daten zum Beispiel mit einem Computer, einem Mobiltelefon oder einem PDA austauschen zu können, also eine so genannte "Long-Range" Kommunikation durchführen zu können.

Weiterhin bezieht sich die Erfindung auf ein System zum Übertragen von Daten mit einem wie oben beschriebenen bevorzugt am oder im Körper eines Patienten tragbaren medizinischen Gerät und einem wie oben beschriebenen externen Steuergerät, wobei diese Geräte nach dem Aktivieren der Sende- und/oder Empfangseinheit auch synchronisiert werden können.

Gemäß einem weiteren Aspekt bezieht sich die Erfindung auf ein Verfahren zum Übertragen von Daten zwischen einem medizinischen Gerät und einem externen Gerät, wobei das sich im Ruhezustand befindende medizinische Gerät von dem externen Gerät vor der Datenübertragung aktiviert wird. Somit kann das medizinische Gerät zum Beispiel in einem wenige oder keine Energie verbrauchenden Ruhezustand bleiben, wenn kein externes Gerät vorhanden oder aktiviert ist, um eine Datenverbindung herzustellen, sodass das medizinische Gerät nur dann Energie für die Datenkommunikation verbraucht, wenn es vorher von einem externen oder Steuergerät aktiviert wurde, welches zur Datenübertragung bereit ist.

Vorzugsweise wird das medizinische Gerät berührungslos zum Beispiel durch ein elektrisches, magnetisches oder elektromagnetisches Feld, Schall und/oder Licht aktiviert. Ebenso ist es auch möglich, das medizinische Gerät durch einen direkten physikalischen Kontakt mit dem Steuergerät zu aktivieren. Vorzugsweise erfolgt die Aktivierung des medizinischen Gerätes nur dann, wenn ein vorgegebener Abstand von zum Beispiel 2 bis 10cm, also zum Beispiel 5 cm unterschritten wurde, wobei es möglich ist, dass zum Beispiel ein Aktivierungsschalter des medizinischen Gerätes eine Kommunikation zwischen diesem und einem externen Gerät wieder abbricht, wenn ein vorgegebener Abstand wieder überschritten wird. Die Aktivierung des medizinischen Gerätes kann auch zeitlich begrenzt sein oder mit der Ausführung einer Aktion des medizinischen Gerätes beendet werden, wie zum Beispiel nach dem Transfer von Messdaten aus dem Speicher eines Sensor über die Sende- und Empfangseinheiten zu dem externen Steuergerät, wobei diese Messdaten nach dem Senden an das externe Gerät oder nach dem Empfang eines Bestätigungssignals über den richtigen Empfang der gesendeten Daten im Speicher des medizinischen Gerätes gelöscht werden können. Bei einer solchen Datenübertragung können zum Beispiel alle Daten, die seit dem letzten Datenaustausch gewonnen wurden, wie zum Beispiel Messwerte mit zugehörigem Erfassungszeitpunkt, übertragen und zum Beispiel gelöscht werden, damit der Speicher des medizinischen Gerätes zur Speicherung neuer Daten zur Verfügung steht.

Vorzugsweise können von dem medizinischen Gerät und/oder dem externen Steuergerät Identifikationscodes gesendet werden, um zu verhindern, dass Daten versehentlich zwischen unterschiedlichen nicht zugeordneten Systemen ausgetauscht werden. Der Identifikationscode kann zum Beispiel Teil eines Funkprotokolls sein und zum Beispiel in einer Systemkomponente fest einprogrammiert sein. Ebenso ist es möglich, dass der Identifikationscode zum Beispiel beim Systemstart frei programmierbar ist und zum Beispiel zwei Komponenten nach Aktivierung durch einen Benutzer miteinander gekoppelt werden können. Weiterhin können bekannte RFID tags zum Beispiel am medizinischen Gerät zusammen mit RFID-Transpondern am Steuergerät zur Identifikation verwendet werden.

Die Erfindung wird nachfolgend anhand bevorzugter Ausführungsbeispiele beschrieben. Es zeigen:
- Figur 1: eine erste Ausführungsform eines erfindungsgemäßen Systems zur Aktivierung eines medizinischen Gerätes durch eine Energieübertragung von einem Steuergerät;
- Figur 2: eine zweite Ausführungsform eines erfindungsgemäßen Systems zur Aktivierung eines medizinischen Geräts über einen Permanent-Magneten;
- Figur 3: eine dritte Ausführungsform eines erfindungsgemäßen Systems mit der Fähigkeit zur "Long-Range" Kommunikation;
- Figur 4: eine vierte Ausführungsform eines erfindungsgemäßen Systems, welches die Übertragung einfacher Signale über größere Entfernungen ermöglicht;
- Figur 5: eine fünfte Ausführungsform eines erfindungsgemäßen Systems, welche einen Datenaustausch zwischen einem medizinischen Gerät und einem Steuergerät ermöglicht; und
- Figur 6: die Ausführungsform aus Figur 5 während des Datenaustauschs zwischen einem medizinischen Gerät und einem Steuergerät.

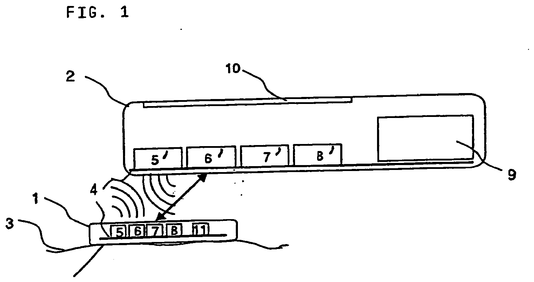

Figur 1 zeigt eine erste Ausführungsform eines erfindungsgemäßen Systems mit einem ambulant auf der Haut 3 eines Anwenders tragbaren medizinischen Gerät 1, welches auf der Haut 3 angebracht oder implantiert sein kann und zum Beispiel eine Pumpe oder ein Sensor ist. Das medizinische Gerät 1 weist eine Induktionsspule 6 zum Aufnehmen einer von einer korrespondierenden Induktionsspule 6' eines externen Steuergeräts 2 durch induktive Kopplung übertragenen Energie auf, wobei die Induktionsspule 6 des medizinischen Gerätes 1 als Aktivierungsschalter für die Sende-/Empfangseinheit 5 und den Prozessor 7 des Gerätes 1 dient, welche von der Induktionsspule 6 mit Strom versorgt werden. Der Prozessor 7 ist mit einem Speicher 8 gekoppelt und kann nach der Aktivierung auch die Sende-/Empfangseinheit 5 ansteuern, um zum Beispiel Mess- oder Konfigurationsdaten aus dem Speicher 8 über die Einheit 5 an die korrespondierende Sende-/Empfangseinheit 5' des externen Steuergeräts 2 zu übertragen oder in Gegenrichtung empfangene Daten, wie zum Beispiel Konfigurationsdaten für das medizinische Gerät 1, zu verarbeiten und/oder in dem Speicher 8 zu speichern. Weiterhin kann in dem medizinischen Gerät 1 optional auch eine Batterie 11 vorgesehen sein, welche zum Beispiel nur die für eine Sensor- oder Pump-Funktion des medizinischen Gerätes 1 erforderliche Energie zur Verfügung stellt, so dass zum Beispiel für die Datenkommunikation keine Energieversorgung im medizinischen Gerät 1 benötigt wird.

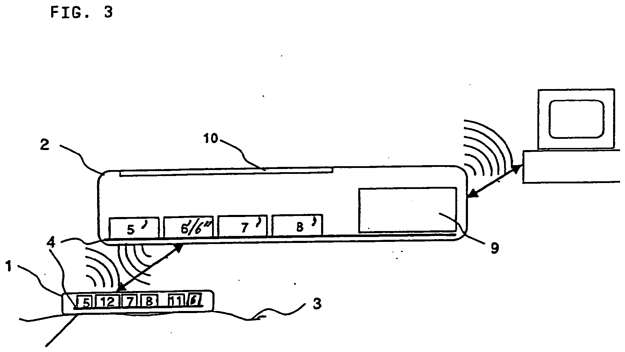

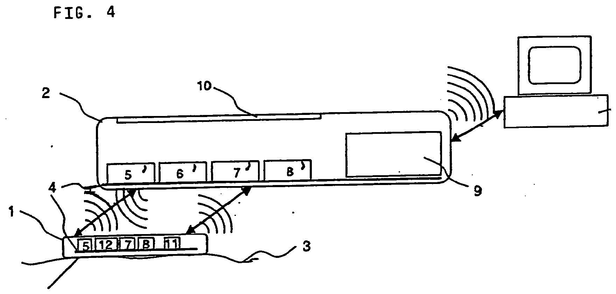

Das externe Steuergerät 2, wie zum Beispiel ein Blutglukosemessgerät, weist ebenso wie das medizinische Gerät 1 einen Prozessor 7' und einen Speicher 8' auf, um zum Beispiel von einem Benutzer über einen Touch-Screen 10 eingegebene Daten und/oder vom medizinischen Gerät 1 erhaltene Daten abzuspeichern oder Daten über die Sende-/Empfangseinheiten 5, 5' an das medizinische Gerät zu übertragen. Weiterhin ist eine Batterie oder ein Akkumulator 9 zur Energieversorgung des externen Steuergerätes 2 vorgesehen. Somit kann eine Kommunikation zwischen dem medizinischen Gerät 1 und dem externen Steuergerät 2 nach der Aktivierung über einen Energietransfer durch die Induktionsspulen 6, 6' hergestellt werden, wobei zum Beispiel nach erfolgter Datenübertragung zwischen diesen Geräten der Speicher des Steuergerätes 2 von einem weiteren Gerät, wie zum Beispiel unter Bezugnahme auf die Figuren 3 und 4 beschrieben, ausgelesen werden kann.

Die elektronischen Baugruppen 5 bis 9 und 11 sind in dem gezeigten Beispiel auf einer Platine 4 angeordnet.

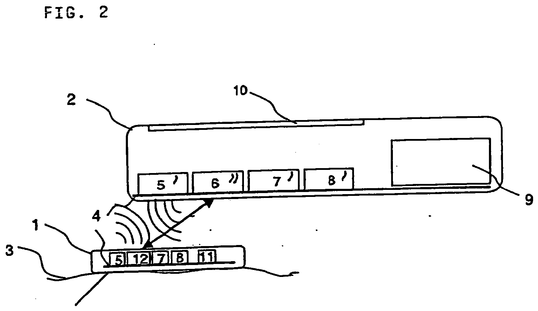

Figur 2 zeigt eine zweite Ausführungsform eines erfindungsgemäßen Systems, wobei im Unterschied zur ersten in Figur 1 gezeigten Ausführungsform ein Permanentmagnet 6' im Steuergerät 2 vorgesehen ist, um einen Magnetschalter 12, wie zum Beispiel einen Reed-Kontakt, des medizinischen Gerätes 1 zu aktivieren, so dass der als Aktivierungsschalter dienende Magnetschalter 12 zum Beispiel die Sende-/Empfangseinheit 5 und/oder den Prozessor 7 des medizinischen Gerätes 1 aktivieren und für eine Datenübertragung freigeben kann.

Figur 3 zeigt eine dritte Ausführungsform eines erfindungsgemäßen Systems, wobei eine Aktivierung oder Datenübertragung des medizinischen Gerätes 1 durch das externe Steuergerät 2 zum Beispiel durch eine Induktionsspule 6' oder einen Permanentmagneten 6" erfolgen kann, welche mit einer Induktionsspule 6 oder einem Magnetschalter 12 des medizinischen Gerätes 1 zusammenwirken. Weiterhin ist das externe Steuergerät 2 so ausgelegt, dass eine Datenverbindung mit einem weiteren externen Gerät 13, wie zum Beispiel mit einem PC, einem Mobiltelefon oder einem PDA, hergestellt werden kann, um Daten von dem Steuergerät 2, welche zum Beispiel an dieses von dem medizinischen Gerät 1 übertragen wurden, an die externe Einheit 13 zu übertragen oder in Gegenrichtung von dieser zu empfangen.

Figur 4 zeigt eine vierte Ausführungsform eines erfindungsgemäßen Systems, wobei zusätzlich zu der in Figur 3 gezeigten dritten Ausführungsform eine Datenkommunikation auch über eine größere Entfernung zwischen dem medizinischen Gerät 1 und dem Steuergerät 2 für bestimmte Signale, wie zum Beispiel kurze Statussignale, möglich ist. Hierzu kann die Sende-/Empfangseinheit 5 des medizinischen Gerätes 1 so ausgelegt sein, dass kurzzeitig unter Einsatz größerer Energie ein zum Beispiel mehrere Meter reichendes Status- oder Requestsignal ausgesandt wird, welches zum Beispiel auch über eine größere Entfernung an das Steuergerät 2 übertragen werden kann, um zum Beispiel einem Benutzer des Steuergerätes 2 zu signalisieren, dass eine Kommunikation mit dem medizinischen Gerät 1 hergestellt werden soll.

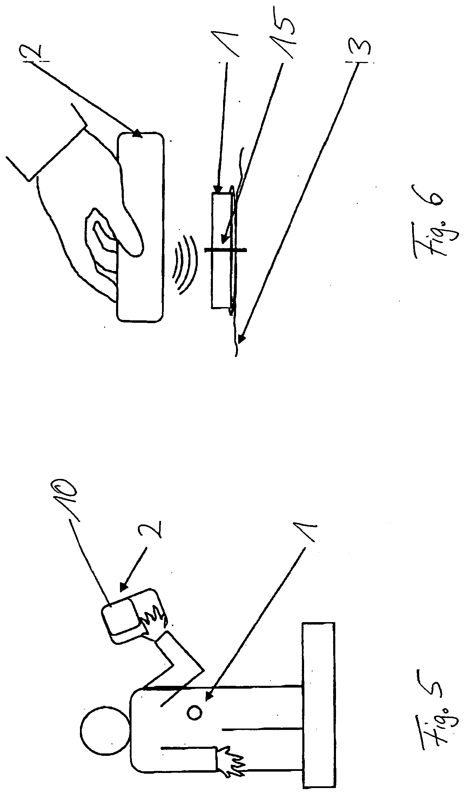

Die Figuren 5 und 6 zeigen eine fünfte Ausführungsform eines erfindungsgemäßen Systems, welches einen Datenaustausch zwischen einem ambulant auf der Haut 3 eines Anwenders tragbaren medizinischen Gerät 1, wie einem Sensor oder einer Pumpe oder Infusionspumpe oder einem so genannten Patch Module, und einem externen, insbesondere von einem Anwender tragbaren oder mobilen, Steuergerät 2, wie einem Blutzucker-Messgerät, ermöglicht. Das medizinische Gerät 1, welches auf der Haut 3 angebracht oder implantiert sein kann, weist zum Beispiel eine Nadel 15 auf, mittels welcher geringe Blutmengen des Anwenders abgenommen werden können. Auch kann das medizinische Gerät 1 subkutan oder unter der Haut 3 des Anwenders angeordnet sein oder kann minimalinvasiv geringe Blutmengen des Anwenders abnehmen. Das medizinische Gerät 1 umfasst eine Recheneinheit, wie einen Prozessor oder Mikroprozessor, einen Speicher, eine Sende-/Empfangseinheit, eine Energiequelle, wie eine Batterie und/oder eine zuvor beschriebene Induktionsspule und/oder einen zuvor beschriebenen Magnetschalter, und eine Messeinheit, wie eine elektronische Schaltung, zum Messen oder Ermitteln mindestens eines physiologischen Parameters des Anwenders, insbesondere des Blutzuckerwerts oder Blutglukosespiegels, anhand der abgenommenen Blutmenge. Die Messeinheit kann optisch arbeiten, so dass das abgenommene Blut mit verschiedenen chemischen Stoffen reagiert, was zu einer Farbänderung eines Testfeldes führt. Diese Farbänderung kann von der Messeinheit erfasst werden und es aus der Dauer und Stärke der Änderung kann der Blutzuckerwert bestimmt werden. Auch kann die Messeinheit amperometrisch arbeiten, so dass durch Messung eines Stromstärke-Verlaufs durch das Blut der Blutzuckerwert bestimmt werden kann.

Das externe Steuergerät 2, wie ein Blutglukosemessgerät oder Datenlogger, weist eine Anzeigevorrichtung 10, insbesondere einen Bildschirm oder ein Display oder einen Touch-Screen, eine Sende-/Empfangseinheit, einen Speicher, eine Energiequelle, wie eine Batterie, und zumindest eine Recheneinheit, insbesondere einen Prozessor oder Mikroprozessor auf. Weiter kann das externe Steuergerät 2 eine in Bezug auf die Figuren 1 bis 4 beschriebenen Aktivierungseinheit, insbesondere eine Induktionsspule oder einen Permanentmagneten, aufweisen. Das Steuergerät 2 kann so ausgebildet sein, dass es anhand von Messdaten den Blutzuckerwert oder den Blutzuckerwertverlauf des Anwenders ermitteln kann, welcher zum Beispiel durch Ausgabe von Zahlenwerten oder eines grafischen Verlaufs oder Diagramms auf der Anzeigevorrichtung 10 dargestellt werden kann.

Wie in Figur 6 gezeigt, kann das externe Steuergerät 2, wie das Blutzucker-Messgerät, in die Nähe oder in Reichweite des medizinischen Geräts 1, wie dem Patch Module, gebracht werden, um einen Datenaustausch oder eine Datenübertragung zwischen dem Steuergerät 2 und dem medizinischen Gerät 1 zu ermöglichen. Vorzugsweise aktiviert dabei die Aktivierungseinheit des Steuergeräts 2 den Aktivierungsschalter des medizinischen Geräts 1, so dass von der Aktivierungseinheit des Steuergeräts 2 Energie auf den Aktivierungsschalter des medizinischen Geräts 1 übertragen werden kann, mittels welcher das medizinische Gerät 1 mit Strom versorgt werden kann. Auch kann beispielsweise die Aktivierungseinheit des Steuergeräts 2 die Energiequelle oder Energieversorgung des medizinischen Geräts 1 aktivieren, so dass das medizinische Gerät 1 nach Aktivierung der Energiequelle mit Strom versorgt werden kann.

Mit dem medizinischen Gerät 1 kann aus dem Blut eines Anwenders ein Blutwert bestimmt werden. Dieser Wert kann beispielsweise mit Seiteninformationen, wie der Uhrzeit der Blutabnahme, von der Sende-/Empfangseinheit des medizinischen Geräts 1 an die Send/Empfangseinheit des Steuergeräts 2 gesendet werden, wenn sich das Steuergerät 2 in ausreichender Nähe des medizinischen Geräts 1 befindet, welche eine Datenübertragung ermöglicht. Aus diesem Blutwert kann in dem Steuergerät 2 von der Recheneinheit des

Steuergeräts 2 der Blutzuckerwert oder Glukosegehalt des Anwenders ermittelt werden und beispielsweise mit den Seiteninformationen in dem Speicher abgespeichert oder auf der Anzeigevorrichtung 10 dargestellt werden.

Auch kann in dem medizinischen Gerät 1 aus dem abgenommenen Blut des Anwenders mittels der Recheneinheit der Blutzuckerwert oder Glukosespiegel des Anwenders ermittelt werden und beispielsweise in der in Figur 6 gezeigten Position oder Anordnung an das Steuergerät 2 beispielsweise mit den Seiteninformationen übertragen werden. Auch können zu verschiedenen Zeitpunkten aufgenommene Blutzuckerwerte mit dem Zeitpunkt oder der Uhrzeit der Abnahme in dem Speicher des medizinischen Geräts 1 gespeichert werden und bei Bedarf von dem externen Steuergerät 2 abgerufen werden. Daten über den ermittelten Blutzuckerspiegel und gegebenenfalls die Seiteninformationen können von der Sende-/Empfangseinheit des medizinischen Geräts 1 an die Sende-/Empfangseinheit des Steuergeräts 2 übertragen werden und können in dem Speicher des Steuergeräts 2 abgespeichert werden oder auf der Anzeigevorrichtung 10 des Steuergeräts 2 als Zahlenwerte oder als Diagram dargestellt werden. Beispielsweise können in dem Steuergerät 2 mehrere zu verschiedenen Zeitpunkten gewonnene Blutzuckerwerte gespeichert und bei Bedarf beispielsweise über der Zeit grafisch dargestellt werden, um ein Blutzuckerspiegel-Diagramm eines Anwenders zu erhalten. Einzelwerte in dem Blutzuckerspiegel-Diagramm können zum Beispiel von dem Anwender mittels des Touch-Screens oder mittels einer anderen Eingabeeinheit ausgewählt werden, welche dann als Zahlenwerte ausgegeben werden können. Insbesondere kann auch unmittelbar nach dem Empfang des von dem medizinischen Gerät ermittelten Blutzuckerwerts der Blutzuckerwert auf der Anzeigevorrichtung 10 des Steuergeräts 2 ausgegeben werden. Auch kann das Steuergerät 2 in Aktivierungs-Reichweite des medizinischen Geräts 1 gehalten werden, so dass die Sende-/Empfangseinheit des Steuergeräts 2 beispielsweise ein Aktivierungssignal an das medizinische Gerät 1 senden kann, worauf das medizinische Gerät 1 aktiviert wird und zum Beispiel eine Blutzuckermessung durchführt und/oder einen ermittelten Blutzuckerwert an das Steuergerät 2 sendet, welcher auf der Anzeigevorrichtung 10 des Steuergeräts 2 ausgegeben werden kann.

Die ermittelten Daten über den Glukosespiegel oder den Glukosespiegelverlauf des Anwenders können über eine an dem Steuergerät 2 angeordnete Schnittstelle, wie einer Infrarot- oder Bluetooth- oder FireWire-Schnittstelle, auf eine externe Recheneinheit, wie einen Computer, übertragen werden und in der Recheneinheit weiterverarbeitet oder ausgegeben oder mit einer Darstellungs- oder Auswertungs-Software dargestellt und/oder ausgewertet werden.

## Patentansprüche

1. Medizinisches System umfassend:
ein medizinisches Gerät (1) zum Einsatz am und/oder im Körper eines Benutzers mit einer Sendeeinheit und/oder Empfangseinheit (5) und einem Aktivierungsschalter (6), und
ein externes Steuergerät (2) mit einer Dateneingabeeinheit, über die der Benutzer Steuersignale eingeben kann, einer Aktivierungseinheit (6', 6") zum Aktivieren des Aktivierungsschalters (6) des medizinischen Geräts (1) und einer Sende- und/oder Empfangseinheit (5'), welche mit der Sendeeinheit und/oder Empfangseinheit (5) des medizinischen Geräts (1) Daten austauschen kann; wobei
der Aktivierungsschalter (6) mit der Sendeeinheit und/oder Empfangseinheit (5) in Verbindung steht, um die Sendeeinheit und/oder Empfangseinheit (5) zu aktivieren, wenn ein Aktivierungssignal des externen Steuergeräts (2) vom Aktivierungsschalter (6) empfangen wurde, **dadurch gekennzeichnet, dass**
der Aktivierungsschalter (6) so ausgebildet ist, dass eine nach der Aktivierung durch das externe Steuergerät (2) hergestellte Kommunikationsverbindung nach einem vorgegebenen Zeitraum, der benötigt wird, um Daten zu übertragen oder eine vorgegebene Aktion auszuführen, wieder unterbrochen wird.

2. Medizinisches System nach Anspruch 1, wobei das medizinische Gerät (1) ein Sensor, ein Glukosesensor, ein Infusionssystem, eine Pumpe, eine Insulin-Pumpe, oder ein Pen ist.

3. Medizinisches System nach Anspruch 1 oder 2, wobei das externe Steuergerät (2) eine Fernbedienung oder ein Glucosemessgerät ist.

4. Medizinisches System nach einem der vorhergehenden Ansprüche, wobei der Aktivierungsschalter (6) eine Induktionsspule, ein Magnetschalter (12), ein Reed-Kontakt, ein Mikrofon oder ein lichtempfindlicher Schalter, eine Fotodiode, ein Sensor, der kapazitive Effekte nutzt, oder ein mechanischer Schalter ist.

5. Medizinisches System nach einem der vorhergehenden Ansprüche, wobei die Aktivierungseinheit (6', 6") eine Induktionsspule (6') und/oder ein Permanentmagnet (6") ist.

6. Medizinisches System nach einem der vorhergehenden Ansprüche, wobei das externe Steuergerät (2) eine Datenausgabeeinheit (10) aufweist.

7. Medizinisches System nach einem der vorhergehenden Ansprüche, wobei das externe Steuergerät (2) eine Schnittstelle zur Kommunikation mit einem elektronischen Gerät, einem PC, Mobiltelefon oder PDA aufweist.

8. Medizinisches System nach einem der vorhergehenden Ansprüche, wobei die hergestellte Kommunikationsverbindung nach dem Zeitraum, der benötigt wird, um die Aktion des Abgebens eines Bolus auszuführen, wieder unterbrochen wird.

9. Medizinisches System nach einem der Ansprüche 1 bis 7, wobei die hergestellte Kommunikationsverbindung nach dem Zeitraum, der benötigt wird, um Messdaten aus einem Speicher (8) des medizinischen Geräts (1) über die Sende- und Empfangseinheiten (5, 5') zu dem externen Steuergerät (2) zu übertragen, wieder unterbrochen wird.

10. Medizinisches System nach Anspruch 9, wobei die Messdaten nach dem Senden oder nach dem Empfang eines Bestätigungssignals über den richtigen Empfang im Speicher (8) des medizinischen Geräts (1) gelöscht werden.

## Claims

1. Medical system comprising:
a medical device (1) for use on and/or in the body of a user with a transmitter unit and/or receiver unit (5) and an activation switch (6) and
an external control device (2) with a data entry unit, via which the user can enter control signals, an activation unit (6', 6") for activating the activation switch (6) of the medical device (1) and a transmission and/or receiving unit (5') which can exchange data with the transmission unit and/or receiving unit (5) of the medical device (1); wherein the activation switch (6) is in connection with the transmission unit and/or receiving unit (5) in order to activate the transmission unit and/or receiving unit (5) when an activation signal of the external control (2) has been received by the activation switch (6), **characterised in that**
the activation switch (6) is configured so that after a predetermined period of time required to transmit data or to implement a predetermined action, a communication connection produced after activation through the external control (2) device is interrupted again.

2. Medical system according to claim 1 wherein the medical device (1) is a sensor, a glucose sensor, an infusion system, a pump, an insulin pump or a pen.

3. Medical system according to claim 1 or 2 wherein the external control device (2) is a remote control or a glucose measuring device.

4. Medical system according to any one of the preceding claims wherein the activation switch (6) is an induction coil, a magnetic switch (12), a reed contact, a microphone or light-sensitive switch, a photodiode, a sensor which uses capacitive effects, or a mechanical switch.

5. Medical system according to any one of the preceding claims wherein the activation unit (6', 6") is an induction coil (6') and/or a permanent magnet (6").

6. Medical system according to any one of the preceding claims wherein the external control device (2) comprises a data output unit (10).

7. Medical system according to any one of the preceding claims wherein the external control device (2) comprises an interface for communication with an electronic device, a PC, mobile telephone or PDA.

8. Medical system according to any one of the preceding claims wherein after the period of time required to implement the action of administering a bolus, the produced communication connection is interrupted again.

9. Medical system according to any one of claims 1 to 7 wherein after the period of time required to transmit measuring data from a memory (8) of the medical device (1) via the transmission and receiving units (5, 5') to the external control device (2), the communication connection is interrupted again.

10. Medical system according to claim 9 wherein after the transmission or after the receipt of a signal confirming correct receipt in the memory (8) of the medical device (1) the measuring data are deleted.

## Revendications

1. Système médical comprenant :
un appareil médical (1) pour utilisation sur et/ou dans un corps d'un utilisateur avec une unité d'émission et/ou une unité de réception (5) et un interrupteur d'activation (6), et
un appareil de commande externe (2) avec une unité d'introduction de données par le biais de laquelle l'utilisateur peut introduire des signaux de commande, une unité d'activation (6",6") pour activer l'interrupteur d'activation (6) de l'appareil médical (1) et une unité d'émission/réception (5'), laquelle peut échanger des données avec l'unité d'émission et/ou l'unité de réception (5) de l'appareil médical (1), l'interrupteur d'activation (6) étant en liaison avec l'unité d'émission et/ou l'unité de réception (5) pour activer l'unité d'émission et/ou l'unité de réception (5), si un signal d'activation de l'appareil de commande externe (2) a été reçu par l'interrupteur d'activation (6), **caractérisé en ce que**
l'interrupteur d'activation (6) est constitué de telle sorte qu'une liaison de communication établie par l'appareil de commande externe (2) est à nouveau interrompue au bout d'une période de temps prédéfinie, qui est nécessaire, pour transmettre des données ou une action prédéfinie.

2. Système médical selon la revendication 1, l'appareil médical (1) étant un capteur, un capteur de glucose, un système de perfusion, une pompe, une pompe à insuline ou un stylet.

3. Système médical selon la revendication 1 ou 2, l'appareil de commande externe (2) étant une télécommande ou un glucomètre.

4. Système médical selon l'une quelconque des revendications précédentes, l'interrupteur d'activation (6) étant une bobine d'induction, un interrupteur magnétique (12), un contact Reed, un microphone ou un interrupteur photosensible, une photodiode, un capteur, qui utilise des effets capacitifs ou un interrupteur mécanique.

5. Système médical selon l'une quelconque des revendications précédentes, l'unité d'activation (6",6") étant une bobine à induction (6') et/ou un aimant permanent (6").

6. Système médical selon l'une quelconque des revendications précédentes, l'appareil de commande externe (2) comportant une unité de sortie de données (10) .

7. Système médical selon l'une quelconque des revendications précédentes, l'appareil de commande externe (2) comportant une interface pour la communication avec un appareil électronique, un ordinateur personnel, un téléphone mobile ou un assistant numérique personnel.

8. Système médical selon l'une quelconque des revendications précédentes, la liaison de communication établie au bout de la période de temps qui est nécessaire, étant à nouveau interrompue pour exécuter l'action d'administration d'un bolus.

9. Système médical selon l'une quelconque des revendications 1 à 7, la liaison de communication établie au bout de la période de temps qui est nécessaire, étant à nouveau interrompue pour transmettre les données de mesure à partir d'une mémoire (8) de l'appareil médical (1) par le biais des unités d'émission et de réception (5,5') à l'appareil de commande externe (2).

10. Système médical selon la revendication 9, les données de mesure étant effacées après l'émission ou la réception d'un signal de confirmation par le biais de la réception correcte dans la mémoire (8) de l'appareil médical (1).
